# EUROPEAN PATENT APPLICATION

(11) **EP 2 127 688 A1**
(43) Date of publication of application: **02.12.2009**
(21) Application number: 08722168.5
(22) Date of filing: 14.03.2008
(51) Int. Cl.: A61L 27/00

(54) **BODY TISSUE FILLING MATERIAL AND METHOD FOR PRODUCTION THEREOF**

(30) Priority: 16.03.2007 JP 2007069160
(71) Applicant: Olympus Corporation, Tokyo 151-0072 (JP); Japan As Represented By National Institute Of Health Sciences, Setagaya-ku Tokyo 158-8501 (JP); Wakitani, Shigeyuki, Joto-ku Osaka-shi Osaka 536-0023 (JP)
(72) Inventor: WAKITANI, Shigeyuki, Osaka-shi Osaka 536-0023 (JP); TAMURA, Tomoaki, Tokyo 151-0072 (JP); HAKAMATSUKA, Yasuharu, Tokyo 151-0072 (JP); TSUCHIYA, Toshie, Tokyo 158-8501 (JP)
(74) Representative: von Hellfeld, Axel
(86) International application number: PCT/JP2008/054772
(87) International publication number: WO 2008/114736

(57) **Abstract**

There are provided a body tissue filling material having excellent engraftability and repairability, and a method for producing such a body tissue filling material in a simple manner. Specifically disclosed are: a body tissue filling material comprising a gelatin porous carrier, to the surface of which a coating having sulfated hyaluronic acid is applied; and a method for producing a body tissue filling material, comprising: a step (S3) of adding dropwise a sulfated hyaluronic acid solution to a gelatin porous carrier to effect impregnation thereof; a step (S4) of freezing the porous carrier impregnated with the sulfated hyaluronic acid solution; and a step (S5) of drying the frozen porous carrier.

## Description

### Technical Field

The present invention relates to a body tissue filling material and a method for producing the same.

### Background Art

Conventionally, there are known base materials for tissue regeneration which comprises a composite of a bioabsorbable hydrophilic material such as porous gelatin and a bioabsorbable polymeric material such as polylactic acid (for example, see Patent Documents 1 and 2).
Patent Document 1:
   PCT International Publication No. WO 96/10426 Pamphlet
Patent Document 2:
   PCT International Publication No. WO 2003/011353 Pamphlet

### Disclosure of Invention

However, the base materials for tissue regeneration disclosed in Patent Document 1 and Patent Document 2 are not always sufficient in the engraftability and repairability of cells due to the issue of biocompatibility and the complexness of implantation of such materials, and thus are incapable of sufficient repair, or may cause inflammation, of the defective part such as a cartilage defective part, which is problematic.

The present invention takes the above situation into consideration with an object of providing a body tissue filling material having excellent engraftability and repairability, and a method for producing such a body tissue filling material in a simple manner.

In order to achieve the above object, the present invention provides the following solutions.
A first aspect of the present invention is a body tissue filling material comprising a gelatin porous carrier, to the surface of which a coating having sulfated hyaluronic acid is applied.
According to the first aspect, because of the action of sulfated hyaluronic acid coated on the surface of the gelatin porous carrier, unstable but variously useful cytokines (such as bFGF, IGF, and HGF) are adsorbed thereto and stabilized, and the effects of these cytokines can be enhanced in parts where they are localized. In addition, sulfated hyaluronic acid promotes the expression of the Wnt gene group via a plasma membrane protein Notch, and thus is capable of promoting the expression of cadherin, connexin, and other intercellular adhesion factors through the β-catenin pathway to thereby particularly promote the differentiation into cartilage. Furthermore, sulfated hyaluronic acid is able to improve the gene expression of IGF binding proteins (IGFNBPs), to define the position of aggregation where the cartilage differentiation occurs, and to suppress the decomposition of IGFs related to the cartilage differentiation so as to thereby promote the cartilage differentiation. By so doing, cell differentiation into cartilage and cell proliferation can be promoted, and the defective part in the body tissue, in particular in cartilage, can be rapidly repaired.

In the first aspect, the sulfonation degree of the sulfated hyaluronic acid is preferably 0.6 or higher.
By so doing, the proliferation potency and the differentiation potency of cartilage cells can be greatly improved.

A second aspect of the present invention is a method for producing a body tissue filling material, comprising: a step of adding dropwise a sulfated hyaluronic acid solution to a gelatin porous carrier to effect impregnation thereof; a step of freezing the porous carrier impregnated with the sulfated hyaluronic acid solution; and a step of drying the frozen porous carrier.
According to the second aspect, the body tissue filling material comprising a gelatin porous carrier, to the surface of which a coating having sulfated hyaluronic acid is applied, can be produced in a simple manner.

In the second aspect, a step of impregnating the gelatin porous carrier with pure water and a step of dehydrating moisture impregnated in the porous carrier may be performed prior to the step of impregnating with the sulfated hyaluronic acid solution.
By so doing, the sulfated hyaluronic acid solution can be infiltrated deep inside the gelatin porous carrier, which makes it possible to produce the body tissue filling material in which sulfated hyaluronic acid is coated even on the inner faces of pores located deep inside the porous carrier.

In the second aspect, the sulfonation degree of the sulfated hyaluronic acid is preferably 0.6 or higher.
By so doing, it becomes possible to produce the body tissue filling material capable of greatly improving the proliferation potency and the differentiation potency of cartilage cells.

The body tissue filling material according to the present invention demonstrates effects of providing excellent engraftability and repairability, and of enabling rapid repair of the defective part. In addition, the method for producing a body tissue filling material according to this embodiment demonstrates an effect of enabling a smiple production of a body tissue filling material which has excellent engraftability and repairability and is capable of rapidly repairing the defective part.

### Brief Description of Drawings

[FIG. 1] FIG. 1 is a flowchart showing a method for producing a body tissue filling material according to one embodiment of the present invention.
[FIG. 2A] FIG. 2A is a graph showing the proliferation potency of cartilage cells with variation of the amount of sulfated hyaluronic acid, where sulfated hyaluronic acid having a sulfonation degree of 0.4 is used for the body tissue filling material according to this embodiment.
[FIG. 2B] FIG. 2B is a graph showing the differentiation potency into cartilage with variation of the amount of sulfated hyaluronic acid, where sulfated hyaluronic acid having a sulfonation degree of 0.4 is used for the body tissue filling material according to this embodiment.
[FIG. 3A] FIG. 3A is a graph similar to FIG. 2A, where sulfated hyaluronic acid having a sulfonation degree of 0.6 is used for the body tissue filling material according to this embodiment.
[FIG. 3B] FIG. 3B is a graph similar to FIG. 2B, where sulfated hyaluronic acid having a sulfonation degree of 0.6 is used for the body tissue filling material according to this embodiment.
[FIG. 4A] FIG. 4A is a graph similar to FIG. 2A, where sulfated hyaluronic acid having a sulfonation degree of 1.0 is used for the body tissue filling material according to this embodiment.
[FIG. 4B] FIG. 4B is a graph similar to FIG. 2B, where sulfated hyaluronic acid having a sulfonation degree of 1.0 is used for the body tissue filling material according to this embodiment.
[FIG. 5A] FIG. 5A is a photograph showing the macroscopic observation of the implantation site with the presence of cells.
[FIG. 5B] FIG. 5B is a photograph showing the macroscopic observation of the implantation site without the presence of cells.
[FIG. 6A] FIG. 6A shows laminagrams showing the histological observation of the cartilage tissue with the presence of cells, stained by toluidine blue and safranin.
[FIG. 6B] FIG. 6B shows laminagrams showing the histological observation of the cartilage tissue without the presence of cells, stained by toluidine blue and safranin.

### Best Mode for Carrying Out the Invention

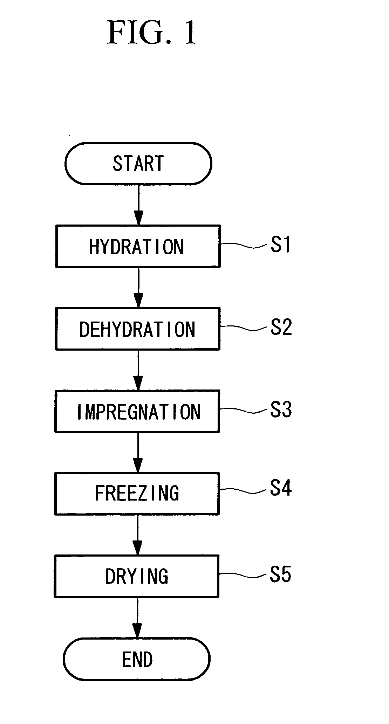

Hereunder is a description of a body tissue filling material and a method for producing the same according to one embodiment of the present invention, with reference to FIG. 1.
The body tissue filling material according to this embodiment comprises a gelatin porous carrier, to the surface of which a coating having sulfated hyaluronic acid is applied. The sulfated hyaluronic acid used herein has its sulfonation degree at 0.6 or higher.

Next is a description of a method for producing a body tissue filling material according to one embodiment of the present invention.
Prior to the production of the body tissue filling material according to this embodiment, tools are sterilized before use by hot air or autoclaving.

In particular, the sponge-shaped gelatin porous carrier should be completely sterilized.
The porous carrier can be prepared by dissolving gelatin with water, adding a cross-linking agent therein, frothing the mixture, and directly thereafter freeze-drying the same. The surplus of the cross-linking agent is removed by washing with water. The water-washed gelatin sponge is air-dried or freeze-dried.
The porous carrier can be prepared by dissolving gelatin with water, frothing the mixture, and directly thereafter freeze-drying the same. The cross-linking can also be introduced into the gelatin sponge by immersing the gelatin sponge in a solution containing a cross-linking agent. In addition, the introduction can also be done by exposing the gelatin sponge to a vapor of the cross-linking agent. The surplus of the cross-linking agent is removed by washing with water. The water-washed gelatin sponge is air-dried or freeze-dried.
The porous carrier can be prepared by dissolving gelatin with water, frothing the mixture, and directly thereafter freeze-drying the same. The cross-linking is introduced into the gelatin sponge by heating under reduced pressure at 100 to 120°C for 1 to 72 hours.
In addition, the introduction can also be done by heating under ordinary pressure at 100 to 180°C for 0.5 to 24 hours.
The pore size of the porous carrier is 50 to 500 µm, and the porosity is about 90%.
Moreover, the sulfated hyaluronic acid solution is prepared by dissolving sulfated hyaluronic acid having a sulfonation degree of 0.6 with pure water at a concentration of 250 µg/mL, and sterilized by filtering.

In the method for producing the body tissue filling material according to this embodiment, firstly, the thus prepared gelatin porous carrier is impregnated with filter-sterilized pure water to effect hydration (S1). Next, the hydrated porous carrier is placed on a filter paper, which has been sterilized by autoclaving and then dried, so as to absorb moisture into the filter paper to thereby effect dehydration (S2).

On the completion of sufficient dehydration of moisture from the porous carrier, a sterilized tetrafluoroethylene resin sheet is placed in a sterilized dish, and the dehydrated porous carrier is placed thereon. Then, the thus prepared sulfated hyaluronic acid solution having a sulfonation degree of 0.6 is slowly added dropwise to the porous carrier so that the solution can reach all over the carrier (S3).

Then, the dish is covered with a lid and sealed in a sterilized pack. The sterilized pack having the dish therein is put in a freezer at -80°C, and frozen over a sufficient time (S4). Then, on the completion of freezing, the dish is quickly taken out from the freezer and put in a freeze dryer before the porous carrier is melt, followed by drying by reducing the pressure (S5).
By so doing, the body tissue filling material according to this embodiment is produced.

According to the thus constituted body tissue filling material of this embodiment, because of the action of sulfated hyaluronic acid coated on the surface of the gelatin porous carrier, unstable but variously useful cytokines (such as bFGF, IGF, and HGF) are adsorbed thereto and stabilized, and the effects of these cytokines can be enhanced in parts where they are localized.
In addition, sulfated hyaluronic acid promotes the expression of the Wnt gene group via a plasma membrane protein Notch, and thus is capable of promoting the expression of cadherin, connexin, and other intercellular adhesion factors through the β-catenin pathway to thereby particularly promote the differentiation into cartilage.

Furthermore, sulfated hyaluronic acid is able to improve the gene expression of IGF binding proteins (IGFNBPs), to define the position of aggregation where the cartilage differentiation occurs, and to suppress the decomposition of IGFs related to the cartilage differentiation so as to thereby promote the cartilage differentiation. By so doing, cell differentiation into cartilage and cell proliferation can be promoted, and the defective part in the body tissue, in particular in cartilage, can be rapidly repaired.

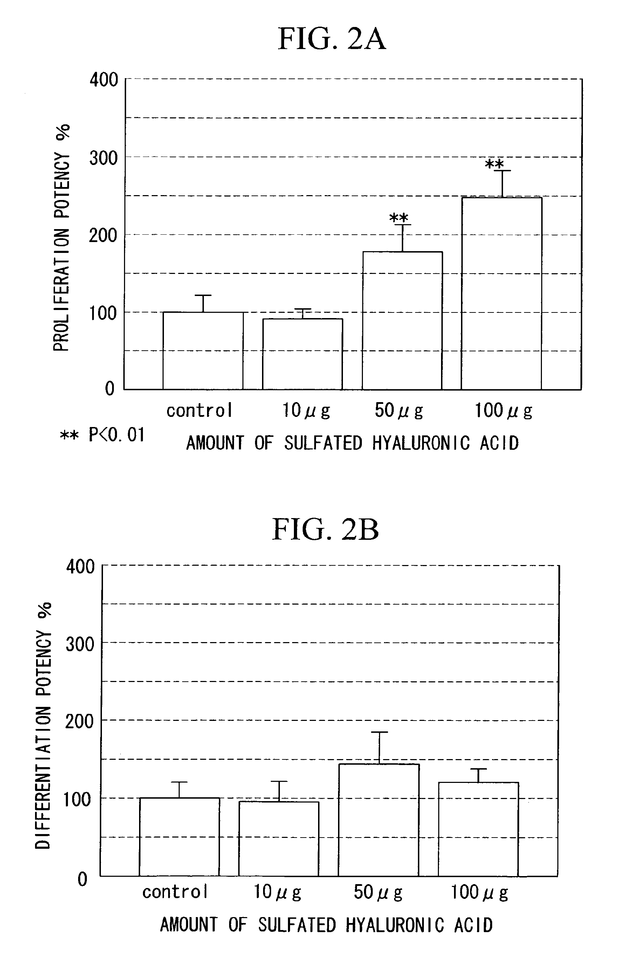

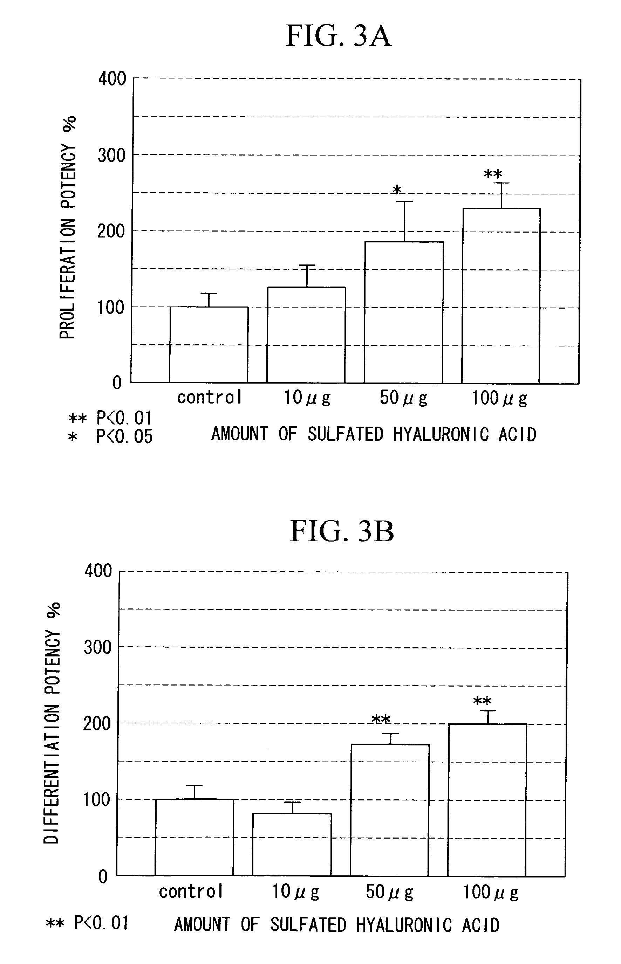

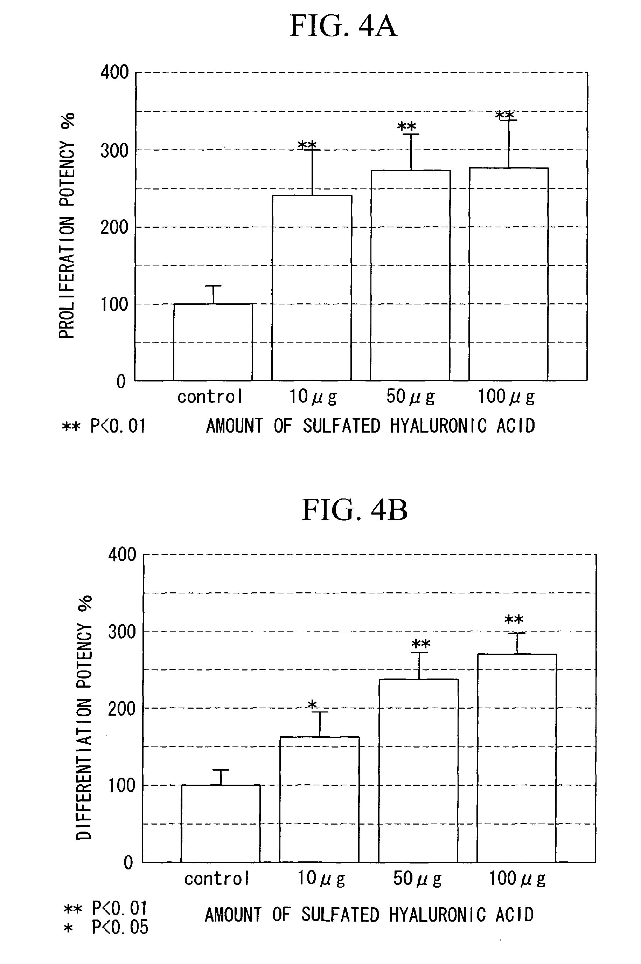

Hereunder is a description of the differences in the proliferation potency and the differentiation potency into cartilage of cartilage cells with variations of the sulfonation degree and the supply amount of sulfated hyaluronic acid, with reference to FIG. 2A to FIG. 4B.
FIG. 2A to FIG. 4B are graphs where the sulfonation degree of sulfated hyaluronic acid was set to 0.4, 0.6, and 1.0 respectively. FIG. 2A, FIG. 3A, and FIG. 4A respectively show the proliferation potency of cells while FIG. 2B, FIG. 3B, and FIG. 4B respectively show the differentiation potency into cartilage. In these graphs, the term "control" shows a case where no sulfated hyaluronic acid was added, while the terms "10 µg", "50 µg", and "100 µg" respectively show the ratios to the control where sulfated hyaluronic acid was added at those amounts.

As for the gelatin porous carrier, a porous material having a diameter of about 12 mm, a thickness of about 3 mm, a pore size of about 50 to 500 µm, and a porosity of about 90% was used. Cartilage cells were seeded in that porous carrier and left standing for 30 minutes.

As a result, according to these FIG. 2A to FIG. 4B, it was found that, when the sulfonation degree was 0.6, the proliferation potency and the differentiation potency into cartilage of cartilage cells were greatly increased with 50 µg or more of sulfated hyaluronic acid. Moreover, it was also found that, when the sulfonation degree was 1.0, the proliferation potency and the differentiation potency into cartilage of cartilage cells were greatly increased irrespective of the amount of sulfated hyaluronic acid.

In this embodiment, sulfated hyaluronic acid was coated on the surface of the porous carrier by impregnating the porous carrier with a sulfated hyaluronic acid solution, and then freeze-drying the same. However, instead of this procedure, the coating may also be carried out by using a binder.
Examples of the binder can include an alkoxysilyl group, an isocyanate group, and 4-methacryloyloxyethyl trimellite anhydride.
In this embodiment, the gelatin sponge is aseptically prepared. However, the gelatin sponge can also be prepared in a non-aseptic manner up to the step before the addition of sulfated hyaluronic acid, if followed by the final sterilization with gamma rays and so forth.

Next is a description of an example in which a body tissue filling material comprising a porous carrier without a coating of sulfated hyaluronic acid was filled in a cartilage defective part.
A cartilage defective part having a diameter of 12 mm and a thickness of 3 mm was made in a miniature pig, and a porous carrier of the same size was produced. The porous carrier was prepared by dissolving gelatin with water, frothing the mixture, and directly thereafter freeze-drying the same. The pore size of the porous carrier was 50 to 500 µm, and the porosity was about 90%.

Regarding the cell suspension, 20 mL of bone marrow fluid of the miniature pig was incubated for 13 days, and bone marrow-derived cells adhered on the petri dish were collected to prepare a cell suspension of 5 × 10⁶ cells/mL.
The porous carrier was once impregnated with a medium, dehydrated, then seeded with one third volume of the cell suspension, and left standing at 37°C. The standing time was 15 minutes, during which the seeding efficiency of 80% or higher was achieved.

The porous carrier seeded with cells was implanted in the cartilage defective part. Since the porous carrier was flexible and swelled by absorbing the cell suspension, the carrier was able to be tightly fitted in the cartilage defective part. Once the carrier was fitted, it would not come off. By so doing, the cartilage defective part was kept in a grafted state without performing a fixing operation by suturing.

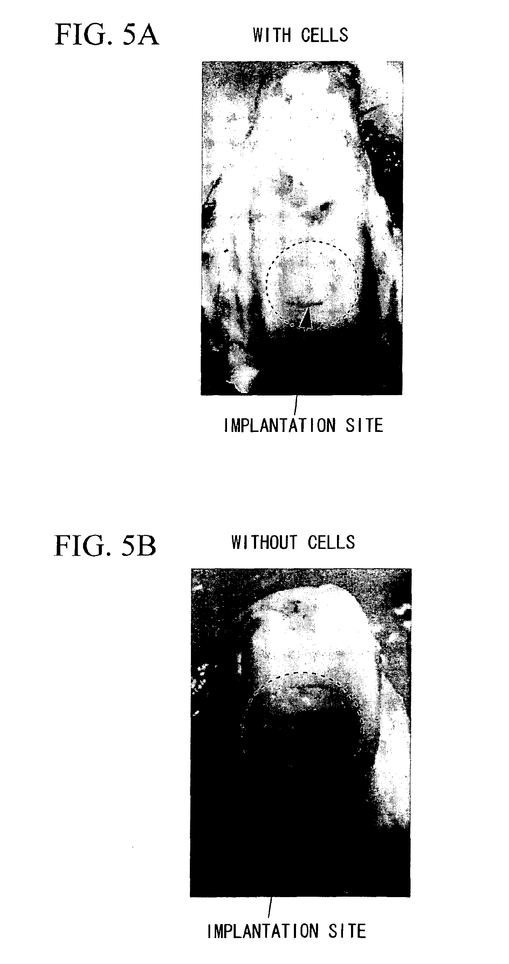

As shown in FIG. 5A and FIG. 5B, according to the macroscopic observation of the implantation site after six months from the implantation, the surface of the grafted part was relatively smooth and the glossiness was no way inferior to the surrounding normal cartilage part. The tissue without the presence of cells was unsmoothly shrunk in the grafted part in FIG. 5B, whereas the tissue with the presence of cells was smooth as compared to the tissue without the presence of cells although the grafted part was slightly rugged in FIG. 5A.

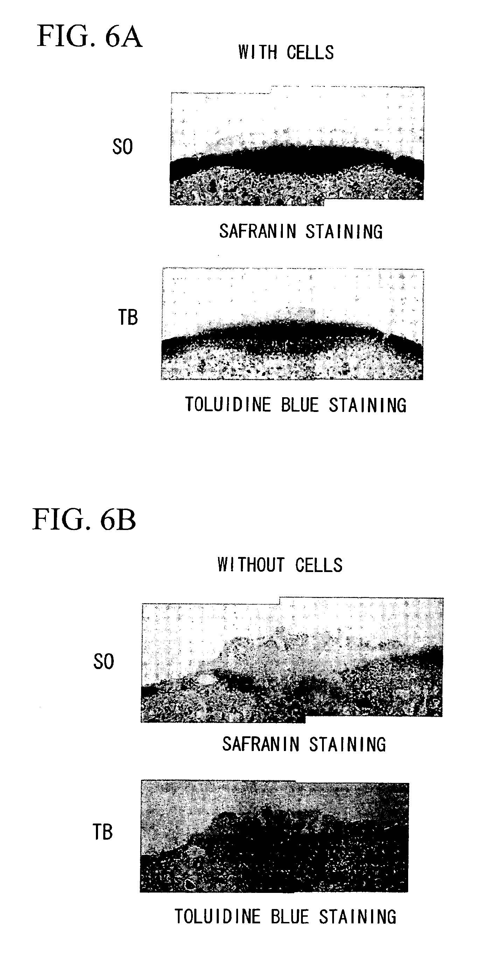

In addition, as shown in FIG. 6A and FIG. 6B, according to the histological observation (toluidine blue and safranin staining), toluidine blue- and safranin O-staining positive parts accounted for the majority. According to FIG. 6A and FIG. 6B where the cartilage tissue is stained, it is understood that the tissue was not properly formed without the presence of cells in FIG. 6B whereas the layered tissue was properly formed with the presence of cells in FIG. 6A.

These observations have confirmed the formation of cartilage in the porous carrier that has been grafted in the cartilage defective part, and also confirmed the repair of the defective part.
The size of the porous carrier is not limited to the same size as the cartilage defective part, and a size slightly larger than the cartilage defective part may also be employed.

As described above, it was shown that the cartilage cell-containing gelatin is effective for repairing cartilage. In addition, as mentioned above, if this gelatin is coated with sulfated hyaluronic acid, it becomes possible to provide a body tissue filling material which is extremely effective for repairing cartilage by the synergetic effect between sulfated hyaluronic acid and cartilage cells, and further to the synergetic effect with cytokines.

## Claims

1. A body tissue filling material comprising a composite of a gelatin porous carrier and sulfated hyaluronic acid.

2. A body tissue filling material comprising a gelatin porous carrier, to the surface of which a coating having sulfated hyaluronic acid is applied.

3. A body tissue filling material according to claim 1, wherein the sulfonation degree of said sulfated hyaluronic acid is 0.6 or higher.

4. A body tissue filling material according to claim 2, wherein the sulfonation degree of said sulfated hyaluronic acid is 0.6 or higher.

5. A method for producing a body tissue filling material, comprising:
a step of adding dropwise a sulfated hyaluronic acid solution to a gelatin porous carrier to effect impregnation thereof;
a step of freezing the porous carrier impregnated with the sulfated hyaluronic acid solution; and
a step of drying the frozen porous carrier.

6. A method for producing a body tissue filling material according to claim 5, wherein
a step of impregnating the gelatin porous carrier with pure water and
a step of dehydrating moisture impregnated in the porous carrier are performed prior to
the step of impregnating with the sulfated hyaluronic acid solution.

7. A body tissue filling material according to claim 5, wherein the sulfonation degree of said sulfated hyaluronic acid is 0.6 or higher.

8. A body tissue filling material according to claim 6, wherein the sulfonation degree of said sulfated hyaluronic acid is 0.6 or higher.

## Amended claims

### Amended claims under Art. 19.1 PCT

**1.** A body tissue filling material comprising a composite of a gelatin porous carrier and sulfated hyaluronic acid.

**2.** A body tissue filling material comprising a gelatin porous carrier, to the surface of which a coating having sulfated hyaluronic acid is applied.

**3.** A body tissue filling material according to claim 1, wherein the sulfonation degree of said sulfated hyaluronic acid is 0.6 or higher.

**4.** A body tissue filling material according to claim 2, wherein the sulfonation degree of said sulfated hyaluronic acid is 0.6 or higher.

**5.** A method for producing a body tissue filling material, comprising:
a step of adding dropwise a sulfated hyaluronic acid solution to a gelatin porous carrier to effect impregnation thereof;
a step of freezing the porous carrier impregnated with the sulfated hyaluronic acid solution; and
a step of drying the frozen porous carrier.

**6.** A method for producing a body tissue filling material according to claim 5, wherein
a step of impregnating the gelatin porous carrier with pure water and
a step of dehydrating moisture impregnated in the porous carrier are performed prior to
the step of impregnating with the sulfated hyaluronic acid solution.

**7.** (Amended) A method for producing a body tissue filling material according to claim 5, wherein the sulfonation degree of said sulfated hyaluronic acid is 0.6 or higher.

**8.** (Amended) A method for producing a body tissue filling material according to claim 6, wherein the sulfonation degree of said sulfated hyaluronic acid is 0.6 or higher.
